# EUROPEAN PATENT SPECIFICATION

(11) **EP 2 999 339 B1**
(45) Date of publication and mention of the grant of the patent: **19.06.2019**
(21) Application number: 14732490.9
(22) Date of filing: 19.05.2014
(51) Int. Cl.: A01N 43/90, A01P 7/00, A61K 9/00, A01P 7/02

(54) **LONG-ACTING SPIRO-ISOXAZOLINE ANTIPARASITIC COMPOSITIONS**
LANG-WIRKENDE SPIRO-OXAZOLIN ZUSAMMENSETZUNGEN GEGEN PARASITEN
COMPOSITIONS ANTIPARASITAIRES À ACTION PROLONGÉE À BASE D'UNE SPIRO-OXAZOLINE

(30) Priority: 20.05.2013 US 201361825177 P
(43) Date of publication of application: 30.03.2016
(73) Proprietor: Zoetis Services LLC, Parsippany, NJ 07054 (US)
(72) Inventor: DE ROSE, Guy, Francis, Kalamazoo, MI 49007 (US); CHUBB, Nathan, Anthony Logan, Kalamazoo, MI 49007 (US); MEEUS, Patrick, F.M., Kalamazoo, MI 49007 (US); MCTIER, Tom, L., Kalamazoo, MI 49007 (US)
(74) Representative: Mannion, Sally Kim
(86) International application number: PCT/US2014/038602
(87) International publication number: WO 2014/189837

(56) References cited:
- WO-A1-00/30449
- WO-A2-2011/157733
- US-A1- 2012 232 026

## Description

### FIELD OF INVENTION

This invention relates to a novel long-acting antiparasitic composition comprising a spiro-azetidine isoxazoline compound, a glycol ether, and at least one veterinarily acceptable solvent, and a method of treating an animal with a parasitic infestation with said composition. The long-acting composition, optionally, comprises at least one additional synergistic veterinary agent.

### BACKGROUND OF THE INVENTION

The present invention relates to a new long-acting veterinary composition comprising a spiro-azetidine isoxazoline for treating an animal with a parasitic infestation, particularly an ectoparasitic infestation. The spiro-azetidine isoxazolines of the instant invention were originally dislosed in WO2012/120399. The present invention provides an improved long-acting (for example, from 2- to 12-months) composition for the treatment of a parasitic infestation in an animal following a single topical dose.

The compounds currently available for parasitic treatment of animals do not always demonstrate good activity, good speed of action, or a long duration of action. Most treatments contain hazardous chemicals that can have serious consequences, including lethality from accidental ingestion. Persons applying these agents are generally advised to limit their exposure. Pet collars and tags have been utilized to overcome some problems, but these are susceptible to chewing, ingestion, and subsequent toxicological affects to the animal. Thus, current treatments achieve varying degrees of success which depend partly on toxicity, method of administration, and efficacy. Currently, some agents are actually becoming ineffective due to parasitic resistance. Hence, there is a need for a stable, long-acting, and effective antiparasitic composition.

The veterinary composition of the present invention provides long-acting efficacy against ectoparasites over other known topical parasiticides. WO 00/30449 discloses stable, long-acting topical parasiticide compositions based on mectins/mycins.

### SUMMARY OF THE INVENTION

The present invention relates to a novel long-acting topical antiparasitic composition. The composition can be used for the treatment and control of parasitic infestations on animals. Further, the invention contemplates the control and prevention of tick borne diseases, for example, bovine anaplasmosis and babesiosis, Lyme disease, epizootic bovine abortion, and theileriosis. Thus, according to the present invention, there is provided an improved long-acting topical composition.

The present invention relates to a long-acting composition comprising 1-(5'-(5-(3,5-dichloro-4-fluorophenyl)-5-(trifluoromethyl)-4,5-dihydroisoxazol-3-yl)-3'H-spiro[azetidine-3,1'-isobenzofuran]-1-yl)-2-(methylsulfonyl)ethanone, or a veterinarily acceptable salt thereof. The more preferred compound is the (S) isomer of 1-(5'-(5-(3,5-dichloro-4-fluorophenyl)-5-(trifluoromethyl)-4,5-dihydroisoxazol-3-yl)-3'H-spiro[azetidine-3,1'-isobenzofuran]-1-yl)-2-(methylsulfonyl)ethanone. The preferred (S)-isomer can be in a crystalline or amorphous solid state form when preparing the long-acting composition.

The composition comprises a spiro-azetidine isoxazoline, a glycol ether, and at least one veterinarily acceptable solvent. In yet another aspect of the present invention, the composition comprises the spiro-azetidine isoxazoline (S)-1-(5'-(5-(3,5-dichloro-4-3,1'-isobenzofuran]-1-yl)-2-(methylsulfonyl)ethanone, a glycol ether, and at least one veterinarily acceptable solvent.

The glycol ether is selected from the group consisting of diethylene glycol monomethylether (DEGMME), diethylene glycol monoethylether (DEGMEE, transcutol), diethylene glycol monobutylether (DEGMBE, butyl digol), dipropyleneglycol monomethyl ether (DPGMME, DPG), dipropyleneglycol monoethyl ether (DPGMEE), and diethylene glycol dimethyl ether (DEGDME). In yet another aspect of the invention, the diglycol is diethylene glycol monobutylether.

The at least one veterinarily acceptable solvent is selected from the group consisting of dimethyl isosorbide, caprylic/capric triglyceride, propylene glycol laurate, isopropyl myristate, oleic acid, eucalyptol, benzyl alcohol, benzyl benzoate, ethanol, propylene glycol caprylate, labrasol, and isopropanol, or any mixture thereof.

In yet another aspect of the invention, the composition further comprises an antioxidant. In yet another aspect of the invention, the antioxidant is selected from butylated hydroxyanisole (BHA), butylated hydroxyltoluene (BHT), propyl gallate, or citric acid, or any mixture thereof. In yet another aspect of the invention, the antioxidant is BHA or BHT.

In yet another aspect of the invention, the composition further comprises a precipitation inhibitor. In yet another aspect of the invention, the precipitation inhibitor is selected from poloxamer F68 and F127, polyvinylpyrrolidones (for example, K-15, K-18, K-20, and the like), alginates, celluloses, and the like, and mixtures thereof.

In yet another aspect of the invention, the composition further comprises at least one additional antiparasitic agent. In yet another aspect of the invention, the additional antiparasitic agent is selected from the group consisting of selamectin, doramectin, moxidectin, abamectin, milbemycin, milbemycin oxime, levamisole, praziquantel, pyrantel, fipronil, an IGR (for example, methoprene, kinoprene, hydroprene, and the like), demiditraz, permethrin, pyrethins, spinosad, and the like, and mixtures thereof.

In yet another aspect of the invention, is the composition for use for treating an animal with a parasitic infestation comprising administering a composition comprising a spiro-azetidine isoxazoline, a glycol ether, and at least one veterinarily acceptable solvent. In yet another aspect of the invention, is the composition for use for treating an animal with a parasitic infestation comprising administering a composition comprising a spiro-azetidine isoxazoline, a glycol ether, at least one veterinarily acceptable solvent, and at least one precipitation inhibitor, and optionally, at least one antioxidant. In yet another aspect of the invention, is the composition for use for treating an animal with a parasitic infestation comprising administering a composition comprising a spiro-azetidine isoxazoline, a glycol ether, at least one veterinarily acceptable solvent, at least one precipitation inhibitor, and at least one antioxidant. In yet another aspect of the invention, is the composition for use for treating an animal with a parasitic infestation comprising administering a composition comprising a spiro-azetidine isoxazoline, a glycol ether, at least one veterinarily acceptable solvent, at least one precipitation inhibitor, at least one antioxidant, and at least one additional veterinary agent.

In yet another aspect of the invention, is an effective amount of the spiro-azetidine isoxazoline. In yet another aspect of the invention, is the composition for use for treating an animal with a parasitic infestation comprising administering a composition comprising an effective amount of (S)-1-(5'-(5-(3,5-dichloro-4-fluorophenyl)-5-(trifluoromethyl)-4,5-dihydroisoxazol-3-yl)-3'H-spiro[azetidine-3,1'-isobenzofuran]-1-yl)-2-(methylsulfonyl)ethanone.

In yet another aspect of the invention, is the composition for use for treating an animal with a parasitic infestation comprising administering a composition comprising an effective amount of (S)-1-(5'-(5-(3,5-dichloro-4-fluorophenyl)-5-(trifluoromethyl)-4,5-dihydroisoxazol-3-yl)-3'H-spiro[azetidine-3,1'-isobenzofuran]-1-yl)-2-(methylsulfonyl)ethanone, wherein the composition further comprises a glycol ether and at least one veterinarily acceptable solvent, and optionally, at least one precipitation inhibitor, at least one antioxidant, and an additional veterinary agent, and any mixture thereof. In yet another aspect of the invention, is the composition for use for treating an animal with a parasitic infestation comprising administering a composition comprising an effective amount of (S)-1-(5'-(5-(3,5-dichloro-4-fluorophenyl)-5-(trifluoromethyl)-4,5-dihydroisoxazol-3-yl)-3'H-spiro[azetidine-3,1'-isobenzofuran]-1-yl)-2-(methylsulfonyl)ethanone, and further comprises at least one additional antiparasitic agent, for example, selamectin.

In yet another aspect of the invention, the animal is a companion animal or livestock. In yet another aspect of the invention, the companion animal is feline, canine, and equine. In yet another aspect of the invention, the companion animal is feline and canine. In yet another aspect of the invention, the companion animal is feline. In yet another aspect of the invention, the companion is canine. In yet another aspect of the invention livestock is ovine, swine, and bovine. In yet another aspect of the invention, livestock is ovine. In yet another aspect of the invention, livestock is bovine. In yet another aspect of the invention, livestock is swine.

In yet another spect of the invention, the parasite is an ectoparasite. In yet another aspect of the invention, the ectoparasite is an acarine or an insect. In yet another aspect of the invention, the acarine is a tick. In yet another aspec of the invention, the acarine is a mite. In yet another aspect of the invention, the insect is a flea, louse, fly, or mosquito. In yet another aspect of the invention, insect is a flea, louse, or fly. In yet another aspect of the invention, insect is a flea.

In yet another aspect of the invention, the long-acting composition is administered at least once every 2-months, 3-months, 4-months, 5-months, 6-months, 7-months, 8-months, 9-months, 10-months, 11-months, or 12-months. In yet another aspect of the invention, the long-acting composition is administered at least once every 2- to 6-months. In yet another aspect of the invention, the long-acting composition is administered at least once every 3-months, 4-months, 5-months, or 6-months. In yet another aspect of the invention, the long-acting composition is administered at least once every 2-months. In yet another aspect of the invention, the long-acting composition is administered at least once every 3-months. In yet another aspect of the invention, the long-acting composition is administered at least once every 4-months. In yet another aspect of the invention, the long-acting composition is administered at least once every 5-months. In yet another aspect of the invention, the long-acting composition is administered at least once every 6-months.

In yet another aspect of the invention, the long-acting composition is administered topically.

### DEFINITIONS

For purposes of the present invention, as described and claimed herein, the following terms and phrases are defined as follows:
"About" when used in connection with a measurable numerical variable, refers to the indicated value of the variable and to all values of the variable that are within the experimental error of the indicated value (e.g., within the 95% confidence interval for the mean) or within 10 percent of the indicated value, whichever is greater.
"Animal" as used herein, unless otherwise indicated, refers to an individual animal, and said individual animal is a mammal. Specifically, mammal refers to a vertebrate animal that is human and non-human, which are members of the taxonomic class Mammalia. Non-exclusive examples of non-human mammals include companion animals and livestock. Non-exclusive examples of a companion animal include: dog (canine), cat (feline), llama, and horse (equine). Preferred companion animals are dog, cat, and horse. More preferred is dog or cat. Non-exclusive examples of livestock include: pigs (porcine), camel, rabbits, goat (caprine), sheep (ovine), deer, elk, cattle (bovine), and bison. Preferred livestock is cattle.
"Infestation", as used herein, unless otherwise indicated, refers to the state or condition of having parasites on the body and/or in the body. Furthermore, the infestation may lead to an infection on or in the animal, which may be microbial, viral, or fungal.
"Long-acting", as used herein, unless otherwise indicated, refers to the duration of time between dosing administration. The duration refers to administration of the long-acting topical composition at least once every 2-months, 3-months, 4-months, 5-months, 6-months, 7-months, 8-months, 9-months, 10-months, 11-months, or 12-months, and includes fractional durations within the aforementioned monthly dosing intervals.
"Parasite(s)", as used herein, unless otherwise indicated, refers to ectoparasites. Ectoparasites are organisms of the Arthropoda phylum (arachnids and insects) which feed through or upon the skin of its host. Preferred arachnids are of the order Acarina (acarines), e.g., ticks and mites. Preferred insects are of the Order Diptera which include biting or myiasis-inducing flies (midges, mosquitos, stable fly, horn fly, blow fly (e.g., cochliomyia), horse fly, sand fly, and the like), Siphonaptera (fleas), and Phthiraptera (lice). Parasites also encompasses the different life stages of the ectoparasite, including eggs, pupae, and larvae which feed on or in the body. Parasite(s) also encumbers endoparasites, parasites that live within the body of its host and include helminths (e.g., trematodes, cestodes, and nematodes) and protozoa.
"Therapeutically effective amount", as used herein, unless otherwise indicated, refers to an amount of one of the spiro-azetidine isoxazolines of the present invention that (i) treat or prevent the particular parasitic infestation, (ii) attenuates, ameliorates, or eliminates one or more symptoms of the particular parasitic infestation, or (iii) prevents or delays the onset of one or more symptoms of the particular parasitic infestation described herein.
"Treatment", "treating", and the like, as used herein, unless otherwise indicated, refers to reversing, alleviating, or inhibiting the parasitic infestation. As used herein, these terms also encompass, depending on the condition of the animal preventing the onset of a disorder or condition, or of symptoms associated with a disorder or condition, including reducing the severity of a disorder or condition or symptoms associated therewith prior to affliction with said infestation. Thus, treatment can refer to administration of the composition of the present invention to an animal that is not at the time of administration afflicted with the parasitic infestation, for example, as prophylactic treatment. Treating also encompasses preventing the recurrence of an infestation or of symptoms associated therewith as well as references to "control" (e.g., kill, repel, expel, incapacitate, deter, eliminate, alleviate, minimize, and eradicate).
"Veterinarily acceptable" as used herein, unless otherwise indicated, suggests that the substance or composition must be compatible chemically and/or toxicologically with the other ingredients comprising the composition and/or the animal being treated therewith. Veterinarily acceptable also encompasses pharmaceutically acceptable.

### BRIEF DESCRIPTION OF THE DRAWINGS

Figure 1. Depicts Flux Permeability.
Figure 2. Depicts Dose Dependent Permeability Flux using Franz Cell Diffusion.
Figure 3. Depicts Dose Constant, Butyl Digol:Dimethyl Isosorbide Flux/Kp Determination.
Figure 4. Depicts Dose Constant Butyl Digol:Oleic Acid Flux/Kp Detemination.
Figure 5. 3-Month Canine Pharmacokinetics

### DETAILED DESCRIPTION

The spiro-azetidine isoxazoline compounds can be synthesized according to procedures described in WO2012/120399.

It is to be understood that the spiro-azetidine isoxazoline compounds of the invention contain an asymmetric carbon (chiral) atom, thus compounds of the invention can exist as two or more stereoisomers. Included within the scope of the present invention are all stereoisomers such as enantiomers (e.g. S and R enantiomers) and diasteromers, all geometric isomers and tautomeric forms of the spiro-azetidine isoxazoline compounds. The spiro-azetidine isoxazolines of the present invention can be racemates, which include the (S) and (R) enantiomers.

The present invention provides for a composition for the treatment of a parasitic infestation in an animal which comprises a veterinarily effective amount of a spiro-azetidine isoxazoline compound. The spiro-azetidine compounds of the present invention is 1-(5'-(5-(3,5-dichloro-4-fluorophenyl)-5-(trifluoromethyl)-4,5-dihydroisoxazol-3-yl)-3'H-spiro[azetidine-3,1'-isobenzofuran]-1-yl)-2-(methylsulfonyl)ethanone and stereoisomers thereof, or a veterinary acceptable salt thereof. The more preferred compound is the (S) enantiomer of 1-(5'-(5-(3,5-dichloro-4-fluorophenyl)-5-(trifluoromethyl)-4,5-dihydroisoxazol-3-yl)-3'H-spiro[azetidine-3,1'-isobenzofuran]-1-yl)-2-(methylsulfonyl)ethanone, which is also referred to herein as Compound 1.

Veterinary compositions suitable for the delivery of compounds of the present invention and methods for their preparation will be readily apparent to those skilled in the art. Such compositions and methods for their preparation may be found, for example, in 'Remington's Pharmaceutical Sciences', 19th Edition (Mack Publishing Company, 1995).

In the present invention, the composition comprises a glycol ether selected from the group consisting of diethylene glycol monomethylether (DEGMME), diethylene glycol monoethylether (DEGMEE), diethylene glycol monobutylether (DEGMBE, butyl digol), dipropyleneglycol monomethyl ether (DPGMME), and diethylene glycol dimethyl ether (DEGDME), and mixtures thereof. A preferred glycol ether is DEGMBE. Another preferred glycol ether is DEGMEE.

In the present invention, the composition comprises at least one veterinarily acceptable solvent selected from the group consisting of dimethyl isosorbide (Arlasolve), caprylic/capric triglyceride, propylene glycol laurate, isopropyl myristate, oleic acid, eucalyptol, benzyl alcohol, benzyl benzoate, ethanol, propylene glycol caprylate, caprylocaproyl macrogol-8 glyceride (labrasol), and isopropanol, or any mixture thereof.

The long-acting composition of the present invention can further comprise an antioxidant. Non-limiting examples of antioxidants include: ascorbic acid, vitamin E (tocopherol), vitamin E derivatives, butylated hydroxanisole (BHA), butylated hydroxytoluene (BHT), propyl gallate, thioglycerol, citric acid, and the like.

The long-acting composition of the present invention comprises a spiro-azetidine isoxazoline, a glycol ether, at least one veterinarily acceptable solvent, or a mixture of more than one veterinarily acceptable solvents as described herein.

The long-acting composition of the present invention comprises 1-(5'-(5-(3,5-dichloro-4-fluorophenyl)-5-(trifluoromethyl)-4,5-dihydroisoxazol-3-yl)-3'H-spiro[azetidine-3,1'-isobenzofuran]-1-yl)-2-(methylsulfonyl)ethanone, a glycol ether, at least one veterinarily acceptable solvent, or a mixture of more than one veterinarily acceptable solvents as described herein.

The long-acting composition of the present invention comprises (S)-1-(5'-(5-(3,5-dichloro-4-fluorophenyl)-5-(trifluoromethyl)-4,5-dihydroisoxazol-3-yl)-3'H-spiro[azetidine-3,1'-isobenzofuran]-1-yl)-2-(methylsulfonyl)ethanone, a glycol ether, at least one veterinarily acceptable solvent, or a mixture of more than one veterinarily acceptable solvents as described herein.

Such compositions are prepared in a conventional manner in accordance with standard medicinal or veterinary practice.

The amounts of these spiro-azetidine isoxazoline compounds are easily determined by a skilled artisan and further depend on the dose amount and dose volume of the final composition. Representative amounts of a veterinarily effective amount of a spiro-azetidine isoxazoline compound ranges from about 0.5 mg/kg to about 50 mg/kg, with a preferred range of about 5 mg/kg to about 40 mg/kg. An even more preferred dose of a spiro-azetidine isoxazoline compound is about 10 mg/kg to about 30 mg/kg. An even more preferred dose of a spiro-azetidine isoxazoline compound is about 15 mg/kg to about 25 mg/kg.

The spiro-azetidine isoxazoline compositions of the present invention are useful as parasiticides for the control and treatment of parasitic infestations in an animal. The veterinary compositions of the present invention have utility as a parasiticide, in particular, as an ectoparasitic. The preferred ectoparasites are acarines and insects. The compositions may, in particular, be used in the fields of veterinary medicine, livestock husbandry, and the maintenance of public health: against acarines and insects which are parasitic upon animals, particularly domestic animals such as dogs, cats, cattle, sheep, goats, horses, llamas, bison, and swine, more particularly cats, dogs, and cattle. Some non-limiting examples of acarine parasites include: ticks (e.g., *Ixodes spp., Rhipicephalus spp., Boophilus spp., Amblyomma spp., Hyalomma spp., Haemaphysalis spp., Dermacentor spp., Ornithodorus spp.,* and the like); and mites (e.g., *Dermanyssus* spp., *Sarcoptes* spp., *Psoroptes* spp., *Eutrombicula* spp., *Chorioptes* spp., *Demodex* spp., and the like). Some non-limiting examples of parasitic insects include: chewing and sucking lice (e.g., *Damalinia spp., Linognathus spp.,* and the like); fleas (e.g., *Siphonaptera spp., Ctenocephalides spp.,* and the like); and flies, mosquitos, and midges (e.g., Order Diptera; *Aedes* spp., *Anopheles* spp., *Tabanidae spp*., *Haematobia spp., Stomoxys spp., Dermatobia spp., Simuliidae spp., Ceratopogonidae spp., Psychodidae spp*., Cochliomyia spp., *Muscidae* spp., *Hypoderma* spp., *Gastrophilus* spp., *Simulium* spp., and the like); true bugs (e.g., Order Hemiptera); cockroaches (*Periplaneta spp, Blatella spp)* and wasps and ants *(Hymenoptera spp).*

The composition of the present invention can also be used for the treatment of endoparasites, for example, heartworms, roundworms, hookworms, whipworms, tapeworms, fluke, and other cestodes and trematodes. The gastrointestinal roundworms include, for example, *Ostertagia ostertagi* (including inhibited larvae), *O*. *lyrata, Haemonchus placei, H. similis, H. contortus, Toxocara canis, T.leonina, T. cati, Trichostrongylus axei, T. colubriformis, T. longispicularis, Cooperia oncophora, C. pectinata, C. punctata, C*. *surnabada* (syn. *mcmasteri*), *C. spatula, Ascaris suum, Hyostrongylus rubidus, Bunostomum phlebotomum, Capillaria bovis, B. trigonocephalum, Strongyloides papillosus, S. ransomi, Oesophagostomum radiatum, O. dentatum, O. columbianum, O. quadrispinulatum, Trichuris* spp., and the like. Other parasites include: hookworms (e.g., *Ancylostoma caninum, A.tubaeforme, A.braziliense, Uncinaria stenocephala*); lungworms (e.g., *Dictyocaulus viviparus and Metastrongylus* spp); eyeworms (e.g., *Thelazia* spp.); parasitic stage grubs (e.g., *Hypoderma bovis, H. lineatum, Dermatobia hominis);* kidneyworms (e.g., *Stephanurus dentatus);* screw worm *(e.g., Cochliomyia hominivorax (larvae);* filarial nematodes of the super-family Filarioidea and the Onchocercidae Family. Non-limiting examples of filarial nematodes within the Onchocercidae Family include the genus Brugia spp. (i.e., *B.malayi, B. pahangi, B. timori,* and the like), Wuchereria spp. (i.e., *W. bancrofti,* and the like), Dirofilaria spp. *(D. immitis, D. repens, D. ursi, D. tenuis, D.spectans, D. lutrae,* and the like), Dipetalonema spp. (i.e., *D reconditum, D. repens,* and the like), Onchocerca spp. (i.e., *O*. *gibsoni, O*. *gutturosa, O*. *volvulus,* and the like), Elaeophora spp. *(E.bohmi, E. elaphi, E. poeli, E. sagitta, E. schneideri,* and the like), Mansonella spp. (i.e., M. *ozzardi, M. perstans,* and the like), and Loa spp. (i.e., *L. loa*). In another aspect of the invention, the composition of the present invention is useful for treating endoparasiticidal infection from filarial nematodes within the genus Dirofilaria (i.e., *D .immitis, D. repens, D. ursi, D. tenuis,* and the like).

The following list of additional veterinary agents together with which the composition of the present invention can be used is intended to illustrate the possible combinations, but not to impose any limitation. Non-limiting examples of additional veterinary agents include: amitraz, arylpyrazoles, amino acetonitriles, anthelmintics (e.g., albendazole, cambendazole, dichlorvos, fenbendazole, flubendazole, mebendazole, octadepsipeptides, oxantel, oxfendazole, oxibendazole, paraherquamide, parbendazole, piperazines, praziquantel, epsiprantel, thiabendazole, tetramisole, triclabendazole, emodepside, levamisole, pyrantel, oxantel, morantel, monepantel, and the like), avermectins (e.g., abamectin, doramectin, emamectin, eprinomectin, ivermectin, moxidectin, selamectin, and the like), milbemycin, milbemycin oxime, DEET, demiditraz, diethylcarbamazine, fipronil, insect growth regulators (e.g., lufenuron, novaluron, hydroprene, kinoprene, methoprene, and the like), metaflumizone, niclosamide, nitenpyram, permethrin, pyrethrins, pyriproxyfen, spinosad, and the like, and mixtures thereof. In certain instances, compositions of the present invention with at least one additional veterinary agent can result in a greater-than-additive effect, for example, synergy (a synergistic effect).

The veterinary compositions of the present invention are of particular value in the control of ectoparasites which are injurious to, or spread or act as vectors of diseases in animals, for example those described herein, and more especially in the control of ticks, mites, lice, fleas, midges and biting, nuisance flies, that may cause, for example, leishmaniasis, demidicosis, Lyme, and borreliosis. They are particularly useful in controlling acarines and insects which feed on the skin or tissue or suck the blood of the animal, for which purpose they may be administered topically.

The spiro-azetidine isoxazoline compound binds tightly to ligand-gated chloride channels, in particular those gated by the neurotransmitter gamma-aminobutyric acid (GABA), thereby blocking pre- and post-synaptic transfer of chloride ions across cell membranes in insects and acarines when exposed by ingestion or contact. This mechanism of action results in lethal uncontrolled activity of the central nervous system of insects and acarines yielding highly efficacious control against said ectoparasite.

The method of treating an animal with a parasitic infestation comprises the administration of the long-acting composition comprising a therapeutically effective amount of a spiro-azetidine isoxazoline compound. Administration is contemplated as dermal administration, wherein dermal administration comprises topical administration by spot-on, pour-on, spray-on, and comb-on methods. The long-acting composition can be topically applied to the animal in need thereof, by administering an effective amount of the composition thereof to the animal at least once every 2-months, 3-months, 4-months, 5-months, 6-months, 7-months, 8-months, 9-months, 10-months, 11-months, or 12-months. The preferred dosing administration is contemplated to be at least once every 4 to 8 months, and more preferrably at least once every 3 to 6 months. Fractional dosing intervals between 2- and 12-months is also contemplated.

The veterinary compositions of the present invention also have value for the treatment and control of the various lifecycle stages of arachnids and insects, including egg, nymph, larvae, juvenile and adult stages.

### EXAMPLES

In the following composition tables, C1 refers to the spiro-azetidine isoxazoline, Compound 1, and SAI represents a different spiro-azetidine isoxazoline compound described herein. Non-limiting veterinarily acceptable compositions are shown below. The amounts are exemplified as % weight/volume (w/v). These amounts can readily be converted to mg/mL and normalized weight %, and liquids as mL/mL and normalized weight %. Amounts for solutions are exemplified as volume/volume percent (v/v%) and is determined by dividing solute volume (mL) by the total volume of solution (mL) times 100.

In the formula examples and tables, the following acronyms are herein described: Captex 355 refers to the medium-chain triglyceride, caprylic/capric triglyceride. PVP-K18 is a polyvinylpyrrolidone with a designated viscosity. Capryol-90 (CP90) is propylene glycol caprylate, also known as 1,2-propanediol monocaprylate. Lauroglycol is propylene glycol laurate, also known as 1,2-propanediol monolaurate. Labrasol (LAB) is a mixture of glyceryl and polyethylene glycol esters (caprylocaproyl macrogol-8 glyceride). Triacetin is glycerol triacetin. Poloxamer F127 is also known as Pluronic F127 and is a di-block copolymer of polyoxyethylene and polyoxypropylene. BHA is butylated hydroxyanisole. BHT is butylated hydroxytoluene. DEGMBE is diethylene glycol monobutyl ether (butyl digol). DEGMEE is diethylene glycol monoethyl ether (Transcutol). Arlasolve (ARL) is dimethyl isosorbide. Butyl digol (BD) is diethylene glycol monobutyl ether (DEGMBE). Tween80 is polysorbate 80. The following acronyms include: C1 is Compound 1, SAI is a spiro-azetidine isoxazoline of Formula 1 or Formula 2, NMP (n-methyl pyrrolidone), OA (oleic acid), BnOH (benzyl alcohol), 2P is 2-pyrrolidone, Span80 is sorbitan oleate, Span20 is sorbitan laurate, C200 is Captex200, C355 is Captex355, C15 PVP is C15 polyvinylpyrrolidone, K29 PVP is K29 polyvinylpyrrolidone, K90 PVP is K90 polyvinylpyrrolidone, EtOH is ethanol, IPA is isopropyl alcohol, IPM is isopropyl myristate, DES is diethyl sebacate, TBAC is tributyl acetocitrate, THFFA is tetrahydro furfuryl alcohol, OZD is 4-decy-1,3-oxazolidin-2-one, NOP is n-octyl pyrrolidone; AZ is azone; DPG is dipropylene glycol monomethyl ether, TRC is transcutol, LG90 is lauroglycol/propylene glycol laurate, and EO is ethyl oleate. Range in the following Formula Examples (1-23) below, is depicted in percent, and in particular, the solids (C1, SAI, BHA, BHT, PVP-K18, poloxomer F127, citric acid, and PVP C15) are measured as w/v% and the remaining liquids are measured as v/v%. The following compositions are non-limiting examples, and include:

**Formula 1**

| Component | Range % | Ideal % |
|---|---|---|
| C1 or SAI | 4-30 | 25 |
| butyl digol | 50-100 | 65 |
| dimethylisosorbide | 5-50 | 10 |

**Formula 2**

| Component | Range % | Ideal % |
|---|---|---|
| C1 or SAI | 4-30 | 25 |
| butyl digol | 50-100 | 62.5 |
| dimethyisosorbide | 5-50 | 7.5 |
| Captex 355 | 2-20 | 5.0 |

**Formula 3**

| Component | Range % | Ideal % |
|---|---|---|
| C1 or SAI | 4-30 | 25.0 |
| butyl digol | 50-100 | 67.3 |
| dimethyisosorbide | 5-50 | 7.5 |
| BHA | 0.01-0.2 | 0.2 |

**Formula 4**

| Component | Range % | Ideal % |
|---|---|---|
| C1 or SAI | 4-30 | 25.0 |
| butyl digol | 50-100 | 66.3 |
| dimethyisosorbide | 5-50 | 7.5 |
| BHA | 0.01-0.2 | 0.2 |
| PVPK18 | 0.1-5.0 | 1.0 |

**Formula 5**

| Component | Range % | Ideal % |
|---|---|---|
| C1 or SAI | 4-30 | 25.0 |
| butyl digol | 50-100 | 66.3 |
| dimethyisosorbide | 5-50 | 7.5 |
| BHA | 0.01-0.2 | 0.2 |
| Poloxamer F127 | 0.1-5.0 | 1.0 |

**Formula 6**

| Component | Range % | Ideal % |
|---|---|---|
| C1 or SAI | 4-30 | 25.0 |
| butyl digol | 50-100 | 67.2 |
| dimethyisosorbide | 5-50 | 7.5 |
| BHA | 0.01-0.2 | 0.2 |
| citric Acid | 0.01-1.0 | 0.1 |

**Formula 7**

| Component | Range % | Ideal % |
|---|---|---|
| C1 or SAI | 4-30 | 25.0 |
| butyl digol | 50-100 | 67.5 |
| isopropyl myristate | 5-30 | 7.5 |

**Formula 8**

| Component | Range % | Ideal % |
|---|---|---|
| C1 or SAI | 4-30 | 25.0 |
| butyl digol | 50-100 | 67.5 |
| oleic acid | 5-30 | 7.5 |

**Formula 9**

| Component | Range % | Ideal % |
|---|---|---|
| C1 or SAI | 4-30 | 25.0 |
| butyl digol | 50-100 | 67.5 |
| 1,8-cineole (eucalyptol) | 5-30 | 7.5 |

**Formula 10**

| Component | Range % | Ideal % |
|---|---|---|
| C1 or SAI | 4-30 | 25.0 |
| butyl digol | 50-100 | 67.5 |
| benzyl alcohol | 5-30 | 7.5 |

**Formula 11**

| Component | Range % | Ideal % |
|---|---|---|
| C1 or SAI | 4-30 | 25.0 |
| butyl digol | 50-100 | 67.5 |
| benzyl benzoate | 5-30 | 7.5 |

**Formula 12**

| Component | Range % | Ideal % |
|---|---|---|
| C1 or SAI | 4-30 | 25.0 |
| butyl digol | 50-100 | 65.0 |
| ethanol | 5-80 | 10 |

**Formula 13**

| Component | Range % | Ideal % |
|---|---|---|
| C1 or SAI | 4-30 | 25.0 |
| butyl digol | 50-100 | 65.0 |
| isopropyl alcohol | 5-80 | 10 |

**Formula 14**

| Component | Range % | Ideal % |
|---|---|---|
| C1 or SAI | 4-30 | 25.0 |
| g-Hexalactone | 50-100 | 67.5 |
| dimethyisosorbide | 5-50 | 7.5 |

**Formula 15**

| Component | Range % | Ideal % |
|---|---|---|
| C1 or SAI | 4-30 | 25.0 |
| propylene carbonate | 50-100 | 67.5 |
| dimethyisosorbide | 5-50 | 7.5 |

**Formula 16**

| Component | Range % | Ideal % |
|---|---|---|
| C1 or SAI | 4-30 | 25.0 |
| tetraglycol | 50-100 | 67.5 |
| dimethyisosorbide | 5-50 | 7.5 |

**Formula 17**

| Component | Range % | Ideal % |
|---|---|---|
| C1 or SAI | 4-30 | 25.0 |
| triacetin | 50-100 | 67.5 |
| dimethyisosorbide | 5-50 | 7.5 |

**Formula 18**

| Component | Range % | Ideal % |
|---|---|---|
| C1 or SAI | 4-30 | 25.0 |
| DEGMEE | 50-100 | 60.0 |
| Capryol 90 | 5-50 | 7.5 |
| Labrasol | 5-50 | 7.5 |

**Formula 19**

| Component | Range % | Ideal % |
|---|---|---|
| C1 or SAI | 4-30 | 25.0 |
| Butyl digol | 50-100 | 45.0 |
| Dimethylisosorbide | 5-50 | 10.0 |
| Lauroglycol | 5-50 | 20.0 |
| BHT | 0.01-0.2 | 0.02 |

**Formula 20**

| Component | Range % | Ideal % |
|---|---|---|
| C1 or SAI | 4-30 | 25.0 |
| Butyl digol | 50-100 | 44.5 |
| Dimethylisosorbide | 5-50 | 10.0 |
| Lauroglycol | 5-50 | 20.0 |
| PVP C15 | 0.1-5.0 | 0.5 |
| BHT | 0.01-0.2 | 0.02 |

**Formula 21**

| Component | Range % | Ideal % |
|---|---|---|
| C1 or SAI | 4-30 | 25.0 |
| DEGMEE | 50-100 | 45.0 |
| Dimethylisosorbide | 5-50 | 10.0 |
| Labrasol | 5-50 | 20.0 |
| BHT | 0.01-0.2 | 0.02 |

**Formula 22**

| Component | Range % | Ideal % |
|---|---|---|
| C1 or SAI | 4-30 | 25.0 |
| DEGMEE | 50-100 | 44.5 |
| Dimethylisosorbide | 5-50 | 10.0 |
| Labrasol | 5-50 | 20.0 |
| PVP C15 | 0.1-5.0 | 0.5 |
| BHT | 0.01-0.2 | 0.02 |

**Formula 23**

| Component | Range % | Ideal % |
|---|---|---|
| C1 or SAI | 4-30 | 25.0 |
| Butyl Digol | 50-100 | 45.0 |
| Dimethylisosorbide | 5-50 | 10.0 |
| Captex 200 | 5-50 | 20.0 |

### BIOLOGICAL

### 6 Month PKPD Study

The spiro-azetidine isoxazoline, (S)-1-(5'-(5-(3,5-dichloro-4-fluorophenyl)-5-(trifluoromethyl)-4,5-dihydroisoxazol-3-yl)-3'H-spiro[azetidine-3,1'-isobenzofuran]-1-yl)-2-(methylsulfonyl)ethanone (Compound 1) was used to conduct an *in-vivo* long-acting efficacy study.

The study assessed the efficacy of a 25 mg/kg (0.1 mL/kg) topical dose of Compound 1 versus control against fleas (*Ctenocephalides felis*) and ticks (*Ixodes scapularis*) on beagle dogs. The formulation was butyl digol:dimethyl isosorbide (90:10 v/v%), selected as it had excellent solubility for Compound 1. Eight male and eight female dogs were acclimated to the testing facility. On Day 0 of the study, each animal received a topical dose of Compound 1 or control (formulation without Compound 1). On Days 56, 89, 117, 145, and 173, animals were artificially infested with about 100 adult unfed fleas. Flea counts were conducted 24 hours post infestation. At each count, all fleas were removed from the dogs. On Days 55, 88, 116, 144, and 172, all dogs were infested with about 50 viable, unfed adult ticks. Tick counts were conducted 48 hours post infestation. At each count, all ticks were removed from the dogs. Geometric mean efficacy results are presented in Table 1, below. Further, some hair was removed from the left or right shoulder from three individual dogs from each treatment group 26 days post dose. The hair from each dog was equally divided and placed into two separate 20mL scintillation vials. Ten female *Rhipicephalus sanguineus* and ten female *Ixodes scapularis* ticks were placed in the vials with the hair. Ticks were evaluated for viability at 2-4 hours, 24 hours, 48 hours and 72 hours post vial infestation. The number of ticks found dead in the vials was reported as the mean value for each group and is presented in Table 2, below.

**Table 1. Geometric Mean Live Tick and Flea Counts and percentage Reductions Following Dosing of Compound 1 on Day 0**

| Group | 57 days | | 90 days | | 118 days | | 146 days | | 174 days | |
|---|---|---|---|---|---|---|---|---|---|---|
| | LT | LF | LT | LF | LT | LF | LT | LF | LT | LF |
| Control | 0.8 | 13.8 | 10.1 | 54.9 | 10.2 | 63.5 | 9.3 | 49.2 | 12.7 | 50.5 |
| C1 | 0.0 | 0.2 | 0.0 | 0.0 | 0.1 | 0.9 | 0.3 | 0.8 | 4.5 | 13 |
| %Reduction | 100 | 98.6 | 100 | 100 | 99.1 | 98.6 | 97.3 | 98.3 | 64.3 | 74.3 |

| | | | | | | | | | | |
|---|---|---|---|---|---|---|---|---|---|---|
| LT = Live Ticks; LF = Live Fleas; C1 = Compound 1 | | | | | | | | | | |

Compound 1, administered in a topical formulation comprising butyl digol and dimethyl isosorbide at 25 mg/kg, provided > 95% control of ticks (*Ixodes capularis*) and fleas (*Ctenocephalides felis*) for 5 months, as measured by reductions in geometric mean counts compared to placebo-treated controls.

**Table 2. Mean Live Tick Counts and Percentage Reductions from Tick Infested Hair Samples**

| Species | Group | # Dead Ticks : Hours Post Infestation | | | |
|---|---|---|---|---|---|
| | | 2-4 | 24 | 48 | 72 |
| *I.scapularis* | Compound 1 | 0 | 3.7 | 7.3 | 9.3 |
| | Control | 0 | 1.0 | 1.0 | 1.3 |
| | % Reduction | 0.0 | 72.7 | 86.4 | 85.7 |
| | | | | | |
| *R.sanguineus* | Compound 1 | 0 | 9.0 | 9.3 | 9.7 |
| | Control | 0 | 0 | 0 | 0 |
| | % Reduction | 0.0 | 100.0 | 100.0 | 100.0 |

The results in Table 2 show that Compound 1 also has contact activity against ticks for at least 26-days following the topical dose.

The 6-month pk/pd study using the vehicle provided robust efficacy for at least 5-months. In view of the pk/pd data, Compound 1 (250 mg/mL) in the vehicle (BD:ARL, 90:10 v/v%) was used as a control to guide subsequent formulation development.

### Formulation Development

To assess and optimize the *in-vitro* permeation characteristics of the spiro-azetidine isoxazoline compounds, Franz diffusion cell screening (FDCS) was employed to assess the novel formulations. FDCS yields flux rates and permeation constants for the formulations through a fixed membrane (e.g., canine-, feline-, and bovine-skin). The flux rate is the amount of drug per unit area per unit time that crosses the membrane, and the permeation constant (Kp) is the flux value normalized for the applied concentration of drug product, generally represented in the log10 form (logKp). A higher flux rate can be correlated to higher *in-vivo* Cmax and AUC.

Canine skin, stored at -20°C for a maximum of 1 year, was thawed, trimmed, and dermatomed to give a thin layer of skin about 0.8-1.5mm in thickness. The skin was mounted onto the Franz diffusion cell and equilibrated with the receptor media, 50:50 v/v% EtOH:Milli-Q water, for 2 to 4 hours. The receptor media was selected to provide sink conditions for Compound 1. Once equilibrated, test formulations were applied to the donor side, generally 50pL/cm². Samples were generally obtained at 12, 24, 30, 36, 42, and 48 hours, but can be obtained at any six timepoints out to 72 hours. A control vehicle of with Compound 1 (250 mg/mL) in butyl digol:dimethyl isosorbide (90:10 v/v%) was included in every FDCS study.

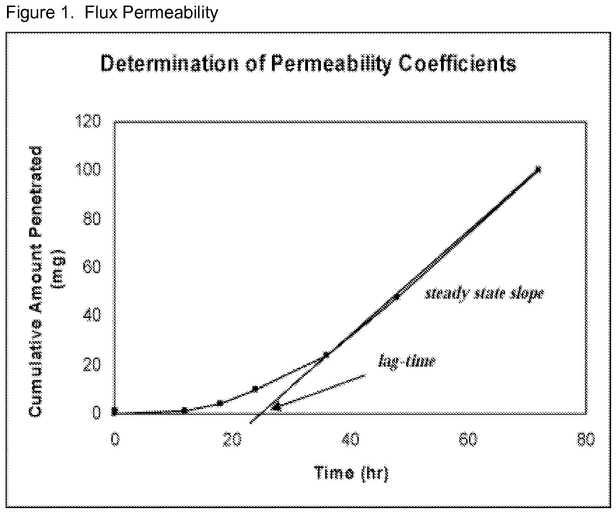

Post experiment samples were stored at 4°C until analysed for Compound 1 concentration by LCMS. Cumulative amount of Compound 1 in ng/cm² was calculated and plotted versus time. The gradient of straight line portion of the graph yields the flux value (ng/cm²/hr) for a given formulation, Figure 1. The flux can be divided by the applied concentration of Compound 1 to yield the permeability constant Kp (cm/hr) - often converted to a log10 scale.

### Example Study 1: Varying Drug Load

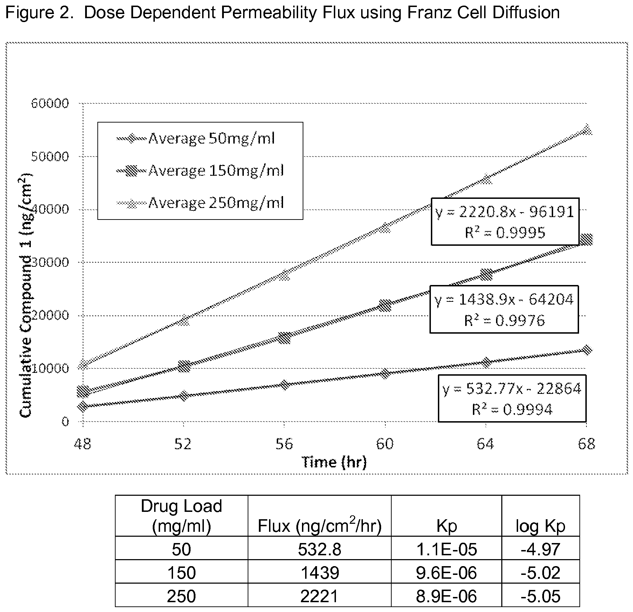

Compound 1 at varying drug loads (50 mg/mL, 150 mg/mL and 250 mg/mL) in butyl digol:dimethyl isosorbide (90:10 v/v%) was assessed in FDCS to determine Flux and Kp and the results are shown in Figure 2, below.

As can be observed in Figure 2, increased drug load correlates to increased flux. The permeability constant (log kp) is unaffected, as would be expected since the same vehicle was used for each group.

### Example Study 2: Dimethyl Isosorbide Titration

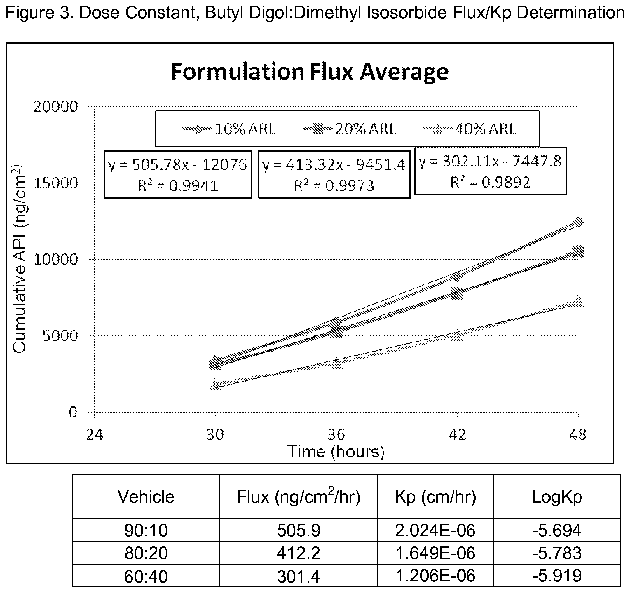

Compound 1 at 250 mg/mL, in varying ratios of butyl digol:dimethyl isosorbide (90:10 v/v%, 80:20 v/v%, and 60:40 v/v%) was assessed in FDCS to determine flux and Kp and the results are shown in Figure 3, below.
The acronym, ARL, refers to Arlasolve, which is dimethyl isosorbide, and API is active pharmaceutical ingredient, and in this instance, Compound 1.

As can be observed in Figure 3, increasing the amount of the solvent, dimethyl isosorbide, induced a small reduction in flux rates and permeation constants. This may indicate that the butyl digol is driving penetration rather than the dimethyl isosorbide component of the formulation.

### Example Study 3: Oleic Acid Titration

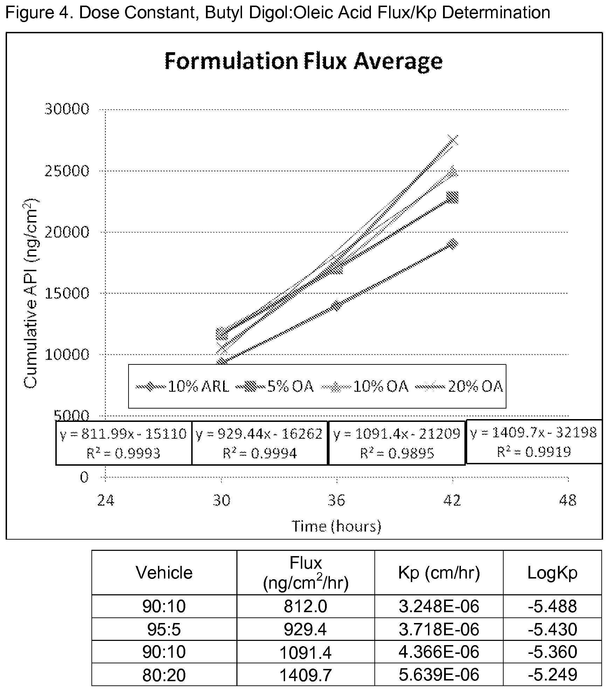

Compound 1 at 250 mg/mL, in varying ratios of butyl digol:oleic acid (95:5 v/v%, 90:10 v/v%, and 80:20 v/v%) was assessed in FDCS to determine flux and Kp and the results are shown in Figure 4, below. The acronym, ARL, refers to Arlasolve, which is dimethyl isosorbide and OA is oleic acid.

As can be observed in Figure 4, flux rates and permeation constants increase with increasing oleic acid levels.

### Results

Mulitple studies were run assessing over 50 novel formulation vehicles. For each study, the Flux of our control vehicle was used to generate an average Flux value (1138.8 ng/cm2/hr). By dividing the average Flux by the experimental Flux generated for each animal skin, a normalization factor for each skin was generated. Data from these studies is presented in Table 3.

**Table 3. Control Vehicle Results and Normalization Factors**

| Study | Skin | Flux (ng/cm²/hr) | Normalisation Factor |
|---|---|---|---|
| Dog | 1 | 441.4 | 2.580 |
| | 2 | 2170.9 | 0.525 |
| | 3 | 226.7 | 5.024 |
| Varying Drug Load | 1 | 2161.0 | 0.527 |
| | 2 | 2124.1 | 0.536 |
| | 3 | 2373.3 | 0.480 |
| Arlasolve Titration | 1 | 542.0 | 2.101 |
| | 2 | 1148.8 | 0.991 |
| | 3 | 321.3 | 3.545 |
| Fatty Ester | 1 | 298.0 | 3.822 |
| | 2 | 1400.7 | 0.813 |
| | 3 | 816.0 | 1.396 |
| IPM Titration | 1 | 765.7 | 1.487 |
| | 2 | 1642.9 | 0.693 |
| | 3 | 2911.4 | 0.391 |
| Oleic Acid Titration | 1 | 172.5 | 6.601 |
| | 2 | 473.1 | 2.407 |
| | 3 | 1790.3 | 0.636 |
| THFFA Titration | 1 | 865.6 | 1.316 |
| | 2 | 469.3 | 2.427 |
| | 3 | 290.9 | 3.916 |
| Alcohol | 1 | 903.8 | 1.260 |
| Pyrrolidone | 1 | 2637.8 | 0.432 |
| Surfactant | 1 | 913.3 | 1.247 |
| Lipophillic Surfactant | 1 | 1277.9 | 0.891 |
| Lauroglycol | 1 | 344.5 | 3.306 |
| IPM-OA | 1 | 1074.3 | 1.060 |
| Pyrrolidone 2 | 1 | 1687.0 | 0.675 |
| Glycol Ether | 1 | 1965.5 | 0.579 |
| Polymer Assessment | 1 | 226.1 | 5.037 |
| Drug Load vs % Lauroglycol | 1 | 868 | 1.312 |

### Normalised Flux and log Kp for Vehicles

In an effort to maximize duration of efficacy, a high and low flux profile was targeted. A "high flux" vehicle generates a higher flux through the skin than the control vehicle leading to an increased Cmax and AUC, achieving higher percent bioavailability and longer duration of action. A "low flux" vehicle generates a lower flux through the skin than the control vehicle leading to a depoting of Compound 1 in the skin. This reservoir serves to maintain efficacious levels of Compound 1 in the plasma extending duration of action.

Multiple vehicles with varying degrees of drug load were assessed to differentiate between low and high flux. The experimental flux value obtained from each vehicle was multiplied by the normalization factor obtained and reported in Table 3, so that each test system could be compared to each other. The test vehicles and flux data is shown in Table 4. In Table 4, log Kp is the permeation constant, drug load is mg/mL, and Flux (ng/cm²/hr) is the normalized average value.

**Table 4. Test Formulations and Relative Flux**

| Study | Drug Load | Vehicle (v/v%) | Flux | log Kp |
|---|---|---|---|---|
| Control | 250 | BD:ARL 90:10 | 1138.80 | -4.341 |
| Drug Load | 150 | BD:ARL 90:10 | 742.67 | -4.527 |
| | 50 | BD:ARL 90:10 | 276.22 | -4.957 |
| Arlasolve Titration | 250 | BD:ARL 80:20 | 1196.60 | -4.320 |
| | 250 | BD:ARL 60:40 | 691.71 | -4.558 |
| Fatty Ester | 250 | BD:IPM 90:10 | 1645.10 | -4.182 |
| | 250 | BD:DES 90:10 | 2177.15 | -4.060 |
| | 250 | BD:TBAC 90:10 | 1991.14 | -4.099 |
| IPM Titration | 250 | BD:IPM 90:10 | 1143.18 | -4.340 |
| | 250 | BD:IPM 80:20 | 1711.06 | -4.165 |
| | 250 | BD:IPM 70:30 | 1938.14 | -4.111 |
| OA Titration | 250 | BD:OA 95:5 | 1445.07 | -4.238 |
| | 250 | BD:OA 90:10 | 1907.31 | -4.118 |
| | 250 | BD:OA 80:20 | 2822.96 | -3.947 |
| THFFA Titration | 250 | BD:THFFA 90:10 | 1522.88 | -4.215 |
| | 250 | BD:THFFA 80:20 | 945.98 | -4.422 |
| | 250 | BD:THFFA 70:30 | 1303.03 | -4.283 |
| Alcohol | 250 | BD:ARL:EtOH 70:10:20 | 1039.52 | -4.381 |
| | 250 | BD:ARL:IPA 70:10:20 | 939.66 | -4.425 |
| | 250 | BD:ARL:BnOH 70:10:20 | 940.24 | -4.425 |
| Pyrrolidone | 250 | BD:2P 80:20 | 709.90 | -4.547 |
| | 250 | BD:NMP 80:20 | 1039.47 | -4.381 |
| | 250 | BD:OZD 80:20 | 1454.99 | -4.235 |
| Surfactant | 250 | BD:ARL:Tween80 70:10:20 | 324.58 | -4.887 |
| | 250 | BD:ARL:LAB 70:10:20 | 350.87 | -4.853 |
| | 250 | BD:ARL:Span20 70:10:20 | 1453.27 | -4.236 |
| Lipophillic Surfactant | 250 | BD:ARL:Span 20 70:10:20 | 1141.82 | -4.340 |
| | 250 | BD:ARL:Span 80 70:10:20 | 1475.63 | -4.229 |
| | 250 | BD:ARL:CP90 70:10:20 | 758.09 | -4.518 |
| | 250 | BD:ARL:LG90 70:10:20 | 2723.54 | -3.963 |
| | 250 | BD:ARL:EO 70:10:20 | 1371.41 | -4.261 |
| | 250 | BD:ARL:C200 70:10:20 | 1853.20 | -4.130 |
| | 250 | BD:ARL:C355 70:10:20 | 1622.34 | -4.188 |
| Lauroglycol Study | 250 | BD:ARL:LG90 85:10:5 | 4276.95 | -3.767 |
| | 250 | BD:ARL:LG90 80:10:10 | 4410.91 | -3.753 |
| | 250 | BD:ARL:LG90 70:10:20 | 7202.60 | -3.540 |
| | 250 | BD:ARL:LG90:C200 70:10:10:10 | 4735.37 | -3.723 |
| | 250 | BD:ARL: LG90:C355 70:10:10:10 | 5544.73 | -3.654 |
| | 250 | BD:ARL: LG90: I PM 70:10:10:10 | 4207.95 | -3.774 |
| IPM:OA Combos | 250 | BD:ARL:IPM:OA 60:10:20:10 | 2173.37 | -4.061 |
| | 250 | BD:ARL:IPM :OA 60:10:10:20 | 2125.66 | -4.070 |
| | 250 | BD:ARL:IPM:OA 80:10:5:5 | 1663.77 | -4.177 |
| | 250 | BD:ARL:IPM:OA 70:10:10:10 | 2513.56 | -3.998 |
| | 250 | BD:ARL:IPM:OA 60:10:15:15 | 1216.64 | -4.313 |
| Pyrollidone 2 | 250 | BD:ARL:2P 85:10:5 | 1888.47 | -4.122 |
| | 250 | BD:ARL:NMP 85:10:5 | 928.48 | -4.430 |
| | 250 | BD:ARL:NOP 85:10:5 | 2371.62 | -4.023 |
| | 250 | BD:ARL:AZ 85:10:5 | 670.95 | -4.571 |
| | 250 | BD:ARL:AZ 80:10:10 | 2202.41 | -4.055 |
| | 250 | BD:ARL:AZ 70:10:20 | 3448.14 | -3.860 |
| Glycol Ether Study | 250 | DPG:ARL 90:10 | 468.51 | -4.727 |
| | 250 | TRC:ARL 90:10 | 207.16 | -5.082 |
| | 250 | TRC:ARL:LG90 70:10:20 | 1286.15 | -4.289 |
| | 250 | TRC:ARL:CP90 70:10:20 | 776.57 | -4.508 |
| | 250 | TRC:ARL:Labrasol 70:10:20 | 103.34 | -5.384 |
| | 250 | TRC:ARL:Tween 80 70:10:20 | 147.63 | -5.229 |
| Polymer Assessment | 250 | BD:ARL:LG90 70:10:20 | 9700.46 | -3.411 |
| | 250 | TRC:ARL:LAB 70:10:20 | 506.80 | -4.693 |
| | 250 | BD:ARL:LG90 60:20:20 | 5336.85 | -3.671 |
| | 250 | TRC:ARL:LAB 60:20:20 | 240.43 | -5.017 |
| | 250 | BD:ARL:LG90 70:10:20+ 2% C15 PVP | 2772.98 | -3.955 |
| | 250 | BD:ARL:LG90 70:10:20 + 2% K29 PVP | 2049.75 | -4.086 |
| | 250 | BD:ARL:LG90 70:10:20 + 2% K90 PVP | 3234.00 | -3.888 |
| Drug Load + % Lauroglycol | 250 | BD:ARL:LG90 70:10:20 | 3384.04 | -3.869 |
| | 200 | BD:ARL:LG90 70:10:20 | 3283.15 | -3.882 |
| | 150 | BD:ARL:LG90 70:10:20 | 3210.18 | -3.891 |
| | 200 | BD:LG90 80:20 | 2597.95 | -3.983 |
| | 200 | BD:ARL:LG90 60:10:30 | 4332.67 | -3.761 |
| | 150 | BD:LG90 80:20 | 3728.01 | -3.826 |
| | 150 | BD:ARL:LG90 60:10:30 | 3912.73 | -3.805 |

Overall, as can be observed in Table 4, the fatty acids, fatty acid esters, and mono esters of propylene glycol with fatty acid elicited the greatest Flux enhancement of Compound 1 through canine skin. A transcutol base vehicle and hydrophilic surfactants Tween 80 and Labrasol had the greatest retarding effect on flux rate through canine skin.

The following formulations in Table 5 were used in a 3-month canine pharmacokinetic study to determine the *in-vitro in-vivo* correlation (IVIVC).

**Table 5. Pharmacokinetic Study Formulations**

| Group | Vehicle (v/v%) | Compound 1 Flux (ng/cm²/hr) | Comment |
|---|---|---|---|
| T01 | Butyl Digol:Arlasolve 90:10 | 1138.8 (n=31) | Control Vehicle |
| T02 | Butyl Digol:Arlasolve:Lauroglycol 70:10:20 | 5752.7 (n=4) | Highest Fluxing |
| T03 | Butyl Digol:Arlasolve:Captex 200 70:10:20 | 1853.2 (n=1) | Higher Flux, spreading |
| T04 | Transcutol:Arlasolve:Labrasol 70:10:20 | 305.1 (n=2) | Lowest Fluxing |

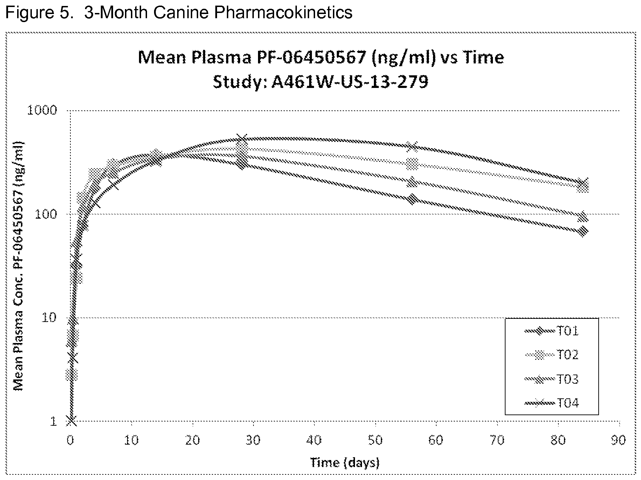

The average flux values in Table 5 were obtained from the individual flux values presented in Table 3. Three month plasma data confirmed good correlation between plasma profiles and the Flux rate values especially at the early timepoints. The plasma Compound 1 profiles for each of the vehicles tested is shown in Figure 5.

The plasma profiles can be viewed in 3 distinct sections. During the first week after dosing both the higher fluxing vehicles (T02, T03) had higher plasma levels than the control vehicle (T01), whilst the low fluxing vehicle (T04) had much lower plasma levels. At day 28 all new vehicles (T02, T03, T04) displayed higher plasma levels than the control (T01), with the lowest flux vehicle showing the highest plasma levels indicating that a drug depot may have been formed in the skin. By the three month time point, day 84, both the high (T02) and low (T04) vehicles had about 3x higher plasma levels than the control vehicle, indicating they should perform better in efficacy studies.

## Claims

1. A long-acting topical composition comprising:
a) a spiro-azetidine isoxazoline which is 1-(5'-(5-(3,5-dichloro-4-fluorophenyl)-5-(trifluoromethyl)-4,5-dihydroisoxazol-3-yl)-3'H-spiro[azetidine-3,1'-isobenzofuran]-1-yl)-2-(methylsulfonyl)ethanone, and stereoisomers thereof, or a veterinary acceptable salt thereof;
b) a glycol ether which is selected from the group consisting of diethylene glycol monomethyl ether, diethylene glycol monoethyl ether, diethylene glycol monobutyl ether, dipropylene glycol monomethyl ether, dipropylene glycol monoethyl ether, diethylene glycol dimethyl ether;
c) at least one veterinarily acceptable solvent selected from the group consisting of dimethyl isosorbide, caprylic/capric triglyceride, propylene glycol laurate, isopropyl myristate, oleic acid, eucalyptol, benzyl alcohol, benzyl benzoate, ethanol, propylene glycol caprylate, caprylocaproyl macrogol-8 glyceride, and isopropanol, or any mixture thereof;
d) optionally, at least one precipitation inhibitor; and
e) optionally, at least one antioxidant.

2. The composition of Claim 1 wherein the spiro-azetidine is (S)-1-(5'-(5-(3,5-dichloro-4-fluorophenyl)-5-(trifluoromethyl)-4,5-dihydroisoxazol-3-yl)-3'H-spiro[azetidine-3,1'-isobenzofuran]-1-yl)-2-(methylsulfonyl)ethanone.

3. The composition of Claim 2 wherein in the glycol ether is a diglycol ether selected from diethylene glycol monoethyl ether or diethylene glycol monobutylether.

4. The composition of Claim 3 further comprising an antioxidant, wherein the antioxidant is selected from butylated hydroxyanisole, butylated hydroxytoluene, propyl gallate, citric acid, or any mixture thereof.

5. A long-acting topical composition according to claim 1 comprising:
a. (S)-1-(5'-(5-(3,5-dichloro-4-fluorophenyl)-5-(trifluoromethyl)-4,5-dihydroisoxazol-3-yl)-3'H-spiro[azetidine-3,1'-isobenzofuran]-1-yl)-2-(methylsulfonyl)ethanone;
b. diethylene glycol monobutylether or diethylene glycol monoethylether;
c. at least one veterinarily acceptable solvent selected from the group consisting of dimethyl isosorbide, caprylic/capric triglyceride, propylene glycol laurate, isopropyl myristate, oleic acid, eucalyptol, benzyl alcohol, benzyl benzoate, ethanol, propylene glycol caprylate, caprylocaproyl macrogol-8 glyceride, and isopropanol, or any mixture thereof;
d. optionally, at least one precipitation inhibitor; and
e. optionally, at least one antioxidant.

6. The composition of claim 5 wherein the solvent is selected from dimethyl isosorbide, propylene glycol laurate, and caprylocaproyl macrogol-8 glyceride, or a mixture thereof, and further comprising at least one precipitation inhibitor.

7. The composition of claim 5 wherein the solvent is selected from isopropyl myristate, oleic acid, eucalyptol, benzyl alcohol, benzyl benzoate, ethanol, and isopropanol, and any mixture thereof, and further comprising at least one precipitation inhibitor.

8. A composition for use in treating an animal with a parasitic infestation comprising administering a long-acting composition comprising:
a) a spiro-azetidine isoxazoline which is 1-(5'-(5-(3,5-dichloro-4-fluorophenyl)-5-(trifluoromethyl)-4,5-dihydroisoxazol-3-yl)-3'H-spiro[azetidine-3,1'-isobenzofuran]-1-yl)-2-(methylsulfonyl)ethanone, and stereoisomers thereof; or _a veterinarily acceptable salt thereof;
b) a glycol ether which is selected from the group consisting of diethylene glycol monomethyl ether, diethylene glycol monoethyl ether, diethylene glycol monobutyl ether, dipropylene glycol monomethyl ether, diethylene glycol dimethyl ether, and mixtures thereof;
c) at least one veterinarily acceptable solvent selected from the group consisting of dimethyl isosorbide, caprylic/capric triglyceride, propylene glycol laurate, isopropyl myristate, oleic acid, eucalyptol, benzyl alcohol, benzyl benzoate, ethanol, propylene glycol caprylate, caprylocaproyl macrogol-8 glyceride, and isopropanol, or any mixture thereof;
d) optionally, at least one precipitation inhibitor; and
e) optionally, at least one antioxidant.

9. The composition for use of Claim 8 wherein the spiro-azetidine isoxazoline is (S)-1-(5'-(5-(3,5-dichloro-4-fluorophenyl)-5-(trifluoromethyl)-4,5-dihydroisoxazol-3-yl)-3'H-spiro[azetidine-3,1'-isobenzofuran]-1-yl)-2-(methylsulfonyl)ethanone.

10. The composition for use of Claim 9 wherein the glycol ether is a diglycol ether selected from diethylene glycol monobutylether or diethylene glycol monoethyl ether.

11. The composition for use of Claim 10 further comprising at least one antioxidant.

12. The composition for use of any one of Claims 8 to 11 wherein the animal is a companion animal or livestock and the composition is administered topically at least once every 3 or more months.

## Patentansprüche

1. Langzeitwirksame topische Zusammensetzung, umfassend:
a) ein Spiro-Azetidinisoxazolin, welches 1-(5'-(5-(3,5-Dichlor-4-fluorphenyl)-5-(trifluormethyl)-4,5-dihydroisoxazol-3-yl)-3'H-spiro[azetidin-3,1'-isobenzo-furan]-1-yl)-2-(methylsulfonyl)ethanon und Stereoisomere davon oder ein veterinär annehmbares Salz davon ist;
b) einen Glycolether, welcher ausgewählt ist aus der Gruppe, bestehend aus Diethylenglycolmonomethylether, Diethylenglycolmonoethylether, Diethylenglycolmonobutylether, Dipropylenglycolmonomethylether, Dipropylenglycolmonoethylether, Diethylenglycoldimethylether;
c) mindestens ein veterinär annehmbares Lösungsmittel, ausgewählt aus der Gruppe, bestehend aus Dimethylisosorbid, Capryl-/Caprinsäuretriglycerid, Propylenglycollaurat, Isopropylmyristat, Oleinsäure, Eukalyptol, Benzylalkohol, Benzylbenzoat, Ethanol, Propylenglycolcaprylat, Caprylocaproylmacrogol-8-glycerid und Isopropanol oder einem beliebigen Gemisch davon;
d) optional mindestens einen Niederschlagshemmer und
e) optional mindestens ein Antioxidans.

2. Zusammensetzung nach Anspruch 1, wobei das Spiro-Azetidin (S)-1-(5'-(5-(3,5-Dichlor-4-fluorphenyl)-5-(trifluormethyl)-4,5-dihydroisoxazol-3-yl)-3'H-spiro[azetidin-3,1'-isobenzo-furan]-1-yl)-2-(methylsulfonyl)ethanon ist.

3. Zusammensetzung nach Anspruch 2, wobei der Glycolether ein Diglycolether, ausgewählt aus Diethylenglycolmonoethylether oder Diethylenglycolmonobutylether, ist.

4. Zusammensetzung nach Anspruch 3, des Weiteren umfassend ein Antioxidans, wobei das Antioxidans ausgewählt ist aus butyliertem Hydroxyanisol, butyliertem Hydroxytoluol, Propylgallat, Citronensäure oder einem beliebigen Gemisch davon.

5. Langzeitwirksame topische Zusammensetzung nach Anspruch 1, umfassend:
a. (S)-1-(5'-(5-(3,5-Dichlor-4-fluorphenyl)-5-(trifluormethyl)-4,5-dihydroisoxazol-3-yl)-3'H-spiro[azetidin-3,1'-isobenzofuran]-1-yl)-2-(methylsulfonyl)ethanon;
b. Diethylenglycolmonobutylether oder Diethylenglycolmonoethylether;
c. mindestens ein veterinär annehmbares Lösungsmittel, ausgewählt aus der Gruppe, bestehend aus Dimethylisosorbid, Capryl-/Caprinsäuretriglycerid, Propylenglycollaurat, Isopropylmyristat, Oleinsäure, Eukalyptol, Benzylalkohol, Benzylbenzoat, Ethanol, Propylenglycolcaprylat, Caprylocaproylmacrogol-8-glycerid und Isopropanol oder einem beliebigen Gemisch davon;
d. optional mindestens einen Niederschlagshemmer und
e. optional mindestens ein Antioxidans.

6. Zusammensetzung nach Anspruch 5, wobei das Lösungsmittel ausgewählt ist aus Dimethylisosorbid, Propylenglycollaurat und Caprylocaproylmacrogol-8-glycerid oder ein Gemisch davon, und des Weiteren umfassend mindestens einen Niederschlagshemmer.

7. Zusammensetzung nach Anspruch 5, wobei das Lösungsmittel ausgewählt ist aus Isopropylmyristat, Oleinsäure, Eukalyptol, Benzylalkohol, Benzylbenzoat, Ethanol und Isopropanol und einem beliebigen Gemisch davon, und des Weiteren umfassend mindestens einen Niederschlagshemmer.

8. Zusammensetzung zur Verwendung bei der Behandlung eines Tiers mit Parasitenbefall, umfassend die Verabreichung einer langzeitwirksamen Zusammensetzung, umfassend:
a) ein Spiro-Azetidinisoxazolin, welches 1-(5'-(5-(3,5-Dichlor-4-fluorphenyl)-5-(trifluormethyl)-4,5-dihydroisoxazol-3-yl)-3'H-spiro[azetidin-3,1'-isobenzo-furan]-1-yl)-2-(methylsulfonyl)ethanon und Stereoisomere davon oder ein veterinär annehmbares Salz davon ist;
b) einen Glycolether, welcher ausgewählt ist aus der Gruppe, bestehend aus Diethylenglycolmonomethylether, Diethylenglycolmonoethylether, Diethylenglycolmonobutylether, Dipropylenglycolmonomethylether, Dipropylenglycolmonoethylether, Diethylenglycoldimethylether, und Gemisches davon;
c) mindestens ein veterinär annehmbares Lösungsmittel, ausgewählt aus der Gruppe, bestehend aus Dimethylisosorbid, Capryl-/Caprinsäuretriglycerid, Propylenglycollaurat, Isopropylmyristat, Oleinsäure, Eukalyptol, Benzylalkohol, Benzylbenzoat, Ethanol, Propylenglycolcaprylat, Caprylocaproylmacrogol-8-glycerid und Isopropanol oder einem beliebigen Gemisch davon;
d) optional mindestens einen Niederschlagshemmer; und
e) optional mindestens ein Antioxidans.

9. Zusammensetzung zur Verwendung nach Anspruch 8, wobei das Spiro-Azetidin (S)-1-(5'-(5-(3,5-Dichlor-4-fluorphenyl)-5-(trifluormethyl)-4,5-dihydroisoxazol-3-yl)-3'H-spiro[azetidin-3,1'-isobenzofuran]-1-yl)-2-(methylsulfonyl)ethanon ist.

10. Zusammensetzung zur Verwendung nach Anspruch 9, wobei der Glycolether ein Diglycolether, ausgewählt aus Diethylenglycolmonobutylether oder Diethylenglycolmonoethylether, ist.

11. Zusammensetzung zur Verwendung nach Anspruch 10, des Weiteren umfassend mindestens ein Antioxidans.

12. Zusammensetzung zur Verwendung nach einem der Ansprüche 8 bis 11, wobei das Tier ein Haustier oder ein Nutztier ist und die Zusammensetzung mindestens einmal alle 3 oder mehr Monate topisch verabreicht wird.

## Revendications

1. Composition topique à action prolongée comprenant :
a) une spiro-azétidine isoxazoline qui est la 1-(5'-(5-(3,5-dichloro-4-fluorophényl)-5-(trifluorométhyl)-4,5-dihydroisoxazol-3-yl)-3'H-spiro[azétidine-3,1'-isobenzofuran]-1-yl)-2-(méthylsulfonyl)éthanone, et les stéréoisomères de celle-ci, ou un sel de celle-ci acceptable dans le domaine vétérinaire ;
b) un éther de glycol qui est sélectionné dans le groupe constitué du diéthylène glycol monométhyl éther, du diéthylène glycol monoéthyl éther, du diéthylène glycol monobutyl éther, du dipropylène glycol monométhyl éther, du dipropylène glycol monoéthyl éther, du diéthylène glycol diméthyl éther ;
c) au moins un solvant acceptable dans le domaine vétérinaire sélectionné dans le groupe constitué du diméthyl isosorbide, du triglycéride caprylique/caprique, du laurate de propylène glycol, du myristate d'isopropyle, de l'acide oléique, de l'eucalyptol, de l'alcool benzylique, du benzoate de benzyle, de l'éthanol, du caprylate de propylène glycol, du caprylocaproyl macrogol-8 glycéride, et de l'isopropanol, ou n'importe quel mélange de ceux-ci ;
d) facultativement, au moins un inhibiteur de précipitation ; et
e) facultativement, au moins un antioxydant.

2. Composition selon la revendication 1 dans laquelle la spiro-azétidine est la (S)-1-(5'-(5-(3,5-dichloro-4-fluorophényl)-5-(trifluorométhyl)-4,5-dihydroisoxazol-3-yl)-3'H-spiro[azétidine-3,1'-iso-benzofuran]-1-yl)-2-(méthylsulfonyl)éthanone.

3. Composition selon la revendication 2 dans laquelle l'éther de glycol est un diglycol éther sélectionné parmi le diéthylène glycol monoéthyl éther ou le diéthylène glycol monobutyléther.

4. Composition selon la revendication 3 comprenant en outre un antioxydant, dans laquelle l'antioxydant est sélectionné parmi l'hydroxyanisole butylé, l'hydroxytoluène butylé, le gallate de propyle, l'acide citrique, ou n'importe quel mélange de ceux-ci.

5. Composition topique à action prolongée selon la revendication 1 comprenant :
a. la (S)-1-(5'-(5-(3,5-dichloro-4-fluorophényl)-5-(trifluorométhyl)-4,5-dihydroisoxazol-3-yl)-3'H-spiro[azétidine-3,1'-isobenzofuran]-1-yl)-2-(méthyl-sulfonyl)éthanone ;
b. le diéthylène glycol monobutyléther ou le diéthylène glycol monoéthyléther ;
c. au moins un solvant acceptable dans le domaine vétérinaire sélectionné dans le groupe constitué du diméthyl isosorbide, du triglycéride caprylique/caprique, du laurate de propylène glycol, du myristate d'isopropyle, de l'acide oléique, de l'eucalyptol, de l'alcool benzylique, du benzoate de benzyle, de l'éthanol, du caprylate de propylène glycol, du caprylocaproyl macrogol-8 glycéride, et de l'isopropanol, ou n'importe quel mélange de ceux-ci ;
d. facultativement, au moins un inhibiteur de précipitation ; et
e. facultativement, au moins un antioxydant.

6. Composition selon la revendication 5 dans laquelle le solvant est sélectionné parmi le diméthyl isosorbide, le laurate de propylène glycol, et le caprylocaproyl macrogol-8 glycéride, ou un mélange de ceux-ci, et comprenant en outre au moins un inhibiteur de précipitation.

7. Composition selon la revendication 5 dans laquelle le solvant est sélectionné parmi le myristate d'isopropyle, l'acide oléique, l'eucalyptol, l'alcool benzylique, le benzoate de benzyle, l'éthanol, et l'isopropanol, et n'importe quel mélange de ceux-ci, et comprenant en outre au moins un inhibiteur de précipitation.

8. Composition pour une utilisation dans le traitement d'un animal ayant une infestation parasitaire comprenant l'administration d'une composition à action prolongée comprenant :
a) une spiro-azétidine isoxazoline qui est la 1-(5'-(5-(3,5-dichloro-4-fluorophényl)-5-(trifluorométhyl)-4,5-dihydroisoxazol-3-yl)-3'H-spiro[azétidine-3,1'-isobenzofuran]-1-yl)-2-(méthylsulfonyl)éthanone, et les stéréoisomères de celle-ci, ou un sel de celle-ci acceptable dans le domaine vétérinaire ;
b) un éther de glycol qui est sélectionné dans le groupe constitué du diéthylène glycol monométhyl éther, du diéthylène glycol monoéthyl éther, du diéthylène glycol monobutyl éther, du dipropylène glycol monométhyl éther, du diéthylène glycol diméthyl éther, et les mélanges de ceux-ci ;
c) au moins un solvant acceptable dans le domaine vétérinaire sélectionné dans le groupe constitué du diméthyl isosorbide, du triglycéride caprylique/caprique, du laurate de propylène glycol, du myristate d'isopropyle, de l'acide oléique, de l'eucalyptol, de l'alcool benzylique, du benzoate de benzyle, de l'éthanol, du caprylate de propylène glycol, du caprylocaproyl macrogol-8 glycéride, et de l'isopropanol, ou n'importe quel mélange de ceux-ci ;
d) facultativement, au moins un inhibiteur de précipitation ; et
e) facultativement, au moins un antioxydant.

9. Composition pour une utilisation selon la revendication 8 dans laquelle la spiro-azétidine isoxazoline est la (S)-1-(5'-(5-(3,5-dichloro-4-fluoro-phényl)-5-(trifluorométhyl)-4,5-dihydroisoxazol-3-yl)-3'H-spiro[azétidine-3,1'-isobenzofuran]-1-yl)-2-(méthyl sulfonyl)éthanone.

10. Composition pour une utilisation selon la revendication 9 dans laquelle l'éther de glycol est un éther de diglycol sélectionné parmi le diéthylène glycol monobutyléther ou le diéthylène glycol monoéthyléther.

11. Composition pour une utilisation selon la revendication 10 comprenant en outre au moins un antioxydant.

12. Composition pour une utilisation selon l'une quelconque des revendications 8 à 11 dans laquelle l'animal est un animal de compagnie ou un bétail et la composition est administrée par voie topique au moins une fois tous les 3 mois ou plus.
